# NEUE EUROPÄISCHE PATENTSCHRIFT

(11) **EP 3 041 454 B2**
(45) Veröffentlichungstag und Bekanntmachung des Hinweises auf die Entscheidung über den Einspruch: **20.12.2023**
(45) Hinweis auf die Patenterteilung: 02.10.2019
(21) Anmeldenummer: 14761318.6
(22) Anmeldetag: 02.09.2014
(51) Int. Cl.: A61K 6/033, A61C 3/025

(54) **PULVERGEMISCH, VERWENDUNG DES PULVERGEMISCHS UND PULVERSTRAHLGERÄT**
POWDER MIXTURE, USE OF THE POWDER MIXTURE AND POWDER JET DEVICE
MÉLANGE DE POUDRES, UTILISATION DU MÉLANGE DE POUDRES ET APPAREIL À JET DE POUDRES

(30) Priorität: 06.09.2013 DE 102013109758
(43) Veröffentlichungstag der Anmeldung: 13.07.2016
(60) Teilanmeldung: 19198493.9
(73) Patentinhaber: Ferton Holding S.A., 2800 Delémont (CH)
(72) Erfinder: DONNET, Marcel, 01630 Saint Jean de Gonville (FR)
(74) Vertreter: Müller Schupfner & Partner Patent- und Rechtsanwaltspartnerschaft mbB
(86) Internationale Anmeldenummer: PCT/EP2014/068622
(87) Internationale Veröffentlichungsnummer: WO 2015/032758

(56) Entgegenhaltungen:
- EP-A1- 2 228 175
- EP-A1- 2 286 786
- EP-A1- 2 572 699
- EP-A2- 0 097 288
- WO-A1-00/53154
- WO-A1-96/29144
- WO-A1-2005/027863
- WO-A1-2007/051546
- WO-A1-2007/137606
- WO-A1-2013/191903
- WO-A1-2013/191903
- DE-A1- 2 134 862
- DE-A1- 10 026 718
- DE-B3- 10 331 583
- DE-U1- 20 009 665
- US-A- 4 612 053
- US-A- 6 054 119
- US-A1- 2006 280 694
- US-A1- 2007 025 891
- US-A1- 2009 175 917
- US-A1- 2010 297 576
- Petersilka et al, J. Clinical Periodontology, 2003, 30, 165-170
- Noji et al, Int J. Abrasive Technology, 2, 2009, 83-96
- Phosphorous World, 2005, p. 194-199

## Beschreibung

Die vorliegende Erfindung betrifft ein Pulvergemisch, insbesondere zur Remineralisierung von Zähnen, ein Pulverstrahlgerät, insbesondere zur Anwendung im Dentalbereich, und eine Verwendung des Pulvergemischs zur Herstellung eines Mittels zur Remineralisierung von Zähnen.

Remineralisation ist ein Begriff aus der Zahnmedizin und bedeutet das Wiedereinlagern von zerstörten bzw. verloren gegangenen Mineralien des Zahnschmelzes nach vorhergehender Demineralisation. Demineralisation ist die Entkalkung durch Verlust von Mineralien. Die Remineralisation beschreibt damit die Wiedereinlagerung von Mineralien in den Zahnschmelz bzw. die Reparatur von kleinsten Schmelzschäden. Die Remineralisation kann im Anfangsstadium die Kariesbildung stoppen oder auch heilen. Dies kann durch gezielte Einnahme von Fluoridpräparaten oder dergleichen ermöglicht werden. Aus dem Stand der Technik sind auch Pulver bekannt, welche zur Bildung eines Remineralisierungsfilms auf die Zähne aufgebracht werden können. Die bekannten Mittel und Verfahren bringen allerdings den Nachteil mit sich, dass die Behandlung oft unangenehm für die Patienten und zudem zeitaufwendig ist. Des Weiteren haften derartige Remineralisierungsfilme auf den Zahnoberflächen nicht.

In der Druckschrift EP 2 572 699 A1 wird ein Pulverstrahlgerät offenbart, das eine Mischeinheit und einer Steuereinheit umfasst, wobei die Mischeinheit ausgelegt ist, aus einem Fluidstrom und/oder einem Luftstrom ein Fluid-Pulver-Gemisch herzustellen. Hierbei weist das Pulverstrahlgerät eine Deponierfunktion auf, mittels welcher ein Remineralisierungsfilm auf den Zähnen gebildet werden kann.

Die Druckschrift US 4 612 053 offenbart ein Pulvergemisch, das als Deponierpulver ein Phosphatpulver aufweist, während im Pulvergemisch aus der Druckschrift US 6 054 119 neben dem als Deponierpulver vorgesehene Phosphatpulver ein Reinigungspulver umfasst.

Die Druckschrift US 2009 / 175917 beschreibt die Verwendung eines Phosphatpulvers aus nanoskaligem Apatit zur Herstellung eines Bleichmittels zur Remineralisierung von Zähnen.

In der Druckschrift DE 10 331 583 ist ein Pulvergemisch offenbart, das als Reinigungspulver Natriumkarbonat enthält.

Es ist also Aufgabe der vorliegenden Erfindung, ein Pulvergemisch, insbesondere zur Remineralisierung von Zähnen, ein Pulverstrahlgerät, insbesondere zur Anwendung im Dentalbereich, ein Verfahren zur Remineralisierung von Zähnen sowie eine Verwendung eines Pulvergemisches zur Herstellung eines entsprechenden Remineralisierungsmittels bzw. in einem Dentalgerät anzugeben, welche die angesprochenen Nachteile beseitigen und ausgelegt sind, einen beständigen Remineralisierungsfilm zu bilden, bei gleichzeitig höchstem Komfort für den Patienten.

Diese Aufgabe wird durch ein Pulvergemisch gemäß Anspruch 1, durch ein Pulverstrahlgerät gemäß Anspruch 6, sowie entsprechende Verwendung gemäß Anspruch 10 gelöst. Weitere Vorteile und Merkmale der Erfindung ergeben sich aus den Unteransprüchen sowie der Beschreibung.

Erfindungsgemäß ist ein Pulvergemisch, insbesondere zur Remineralisierung von Zähnen, dadurch gekennzeichnet, dass das Pulvergemisch ein Deponierpulver aufweist, welches ausgelegt ist, an Zahnoberflächen zu haften. Insbesondere ist das Pulvergemisch ausgelegt, einen Remineralisierungsfilm auf den Zahnoberflächen zu bilden. Mit Vorteil kann dadurch entkalkter Zahnschmelz durch Einlagerung von neuem Kalziumphosphat wieder angereichert und gehärtet werden. Des Weiteren sind dadurch die unter Umständen freiliegenden Dentinkanälchen (Dentintubuli) wieder verschließbar. Die Dentintubuli leiten äußere Reize wie heiß, kalt, süß, sauer oder Berührungen an den Zahnnerv weiter. Die Folge sind sensible und überempfindliche Zähne. Gemäß der hydrodynamischen Theorie nach Brännström (1966) ist dies auf eine Flüssigkeitsverschiebung in den Dentinkanälchen zurückzuführen, die durch die Applikation von Reizen ausgelöst wird. Vorteilhafterweise können durch den Re- bzw. Demineralisierungsfilm offen liegende Dentinkanälchen wieder verschlossen werden. Das Pulvergemisch ist also auch ausgelegt für eine Desensibilisierung der Zähne. Im Folgenden wird aus Gründen der Übersichtlichkeit immer von einem Remineralisierungsfilm gesprochen, was nicht ausschließt, dass es sich dabei sozusagen auch um einen Desensibilisierungsfilm handeln kann.

Zweckmäßigerweise weist der Remineralisierungsfilm eine Dicke von etwa 100 nm bis 2 µm auf. Ein derartiger Film bzw. eine derartige Schicht ist ausreichend, um offen liegende Dentinkanälchen zu schließen und einen Remineralisierungsfilm auf den Zähnen zu bilden, welcher vorteilhafterweise unter anderem Phosphat und Kalzium für Remineralisierungsprozesse bereitstellt. Ein derartiger Remineralisierungsfilm lindert die Schmerzen der Patienten sofort. Eine langwierige Behandlung ist nicht notwendig. Zweckmäßigerweise handelt es sich um ein Pulvergemisch, also um eine Mischung, welche zumindest einen Bestandteil umfasst, wobei einer der Bestandteile mit Vorteil das Deponierpulver ist, welches im Folgenden noch genauer spezifiziert wird. Vorteilhafterweise kann das Pulvergemisch in einem aus dem Stand der Technik bekannten Pulverstrahlgerät, wie es zur Zahnreinigung verwendet wird, eingesetzt werden. Derartige Pulverstrahlgeräte weisen zweckmäßigerweise eine Mischkammer auf, in welcher ein Pulver eingefüllt werden kann, um dann, mit einem Fluid, insbesondere mit Wasser, und Luft vermischt auf die Zähne aufgebracht bzw. aufgesprüht zu werden.

Zweckmäßigerweise ist das Pulvergemisch dadurch gekennzeichnet, dass das Pulvergemisch ein Trägerpulver aufweist, damit es mittels eines Pulverstrahls auf den Zahn aufgebracht werden kann. Das Trägerpulver ist mit Vorteil ein Reinigungspulver, insbesondere Natriumbikarbonat, Glycin, Alditole, wie Erythritol, Kalziumkarbonat oder auch eine Mischung der genannten Stoffe. Bevorzugt erfüllt das Trägerpulver sozusagen eine Transportfunktion für das Deponierpulver. Für das feine Deponierpulver müsste für ein herkömmliches Pulverstrahlgerät eine eigene Düse oder Mischvorrichtung gebaut werden, da die sehr kleinen Partikel des Deponierpulvers sehr schlecht fliegen bzw. sich in einer Mischkammer bzw. Mischvorrichtung nur schwer aufwirbeln lassen. Ein Aufbringen auf die Zahnoberflächen ist daher äußerst schwierig.

Mit Vorteil umfasst daher das Pulvergemisch das Trägerpulver. Das Deponierpulver kann auf dem Trägerpulver zweckmäßigerweise haften, wodurch das Trägerpulver beim Aufbringen des Remineralisierungsfilms sozusagen als Trägermedium für das Deponierpulver verwendet werden kann. Mit Vorteil ist die Oberflächenstruktur des Trägerpulvers bzw. der Partikel des Trägerpulvers derart ausgelegt, dass sich das Deponierpulver anlagern kann. Diese Anlagerung findet bevorzugt bereits bei der Herstellung bzw. Mischung des Pulvergemisches statt und wird während der Lagerung, beispielsweise in einem geeigneten Behältnis oder in der Mischkammer des Pulverstrahlgeräts, aufrechterhalten. Bevorzugt ist die Anlagerung derart ausgelegt, dass sie auch dann, wenn das Pulvergemisch auf die Zähne aufgebracht wird, insbesondere also während des Fluges des Pulvergemisches, aufrechterhalten wird. Mit Vorteil haftet das Deponierpulver auf dem Trägerpulver. Ebenfalls mit Vorteil ist die Oberflächenstruktur der Partikel des Trägerpulvers derart zerklüftet, dass sich das Deponierpulver ein- bzw. anlagern kann. Zum anderen kann das Trägerpulver, insbesondere während des Aufbringens auf die Zähne, eine Windschattenfunktion bereitstellen. Diese kann dem Deponierpulver beste Flugbedingungen ermöglichen. Erreicht wird dies erfindungsgemäß durch eine durchschnittliche Partikelgröße des Trägerpulvers, welche größer ist als eine durchschnittliche Partikelgröße (bzw. Agglomeratgröße) des Deponierpulvers. Mit Vorteil ist damit ein Pulvergemisch bereitgestellt, welches optimal auf die Zahnoberflächen aufbringbar ist und dort auch optimal und lang andauernd haftet.

Mit Vorteil ist das Trägerpulver auch ein Reinigungspulver. Vorteilhafterweise stellt das Pulvergemisch also neben einer Deponierfunktion auch eine Reinigungsfunktion bereit. Ein vorheriges Reinigen der Zahnoberflächen bzw. der Zähne ist dabei besonders vorteilhaft oder unter Umständen sogar notwendig, damit die Dentinkanälchen frei liegen und dann sauber verschlossen werden können. Von großem Vorteil ist es daher, dass das Pulvergemisch eine Deponierfunktion und eine Reinigungsfunktion bereitstellt, da damit zwei Behandlungsmethoden, nämlich das Reinigung und das Deponieren des Pulvergemisches mit ein und demselben Pulvergemisch bzw. auch mit ein und demselben (Pulverstrahl-)Gerät möglich sind.

Das erfindungsgemäße Pulvergemisch zeichnet sich weiter dadurch aus, dass das Reinigungspulver eine mittlere Teilchengröße von zwischen 10 und 100 µm, bevorzugt zwischen 15 und 50 µm, besonders bevorzugt etwa 45 µm aufweist. Falls im subgingivalen Bereich gearbeitet werden soll, so sollte die mittlere Teilchengröße nicht größer ist als 45 µm, bevorzugt nicht größer ist als 35 µm sein, besonders bevorzugt zwischen 10 µm und 30 µm liegen.

Zweckmäßigerweise ist das Pulvergemisch dadurch gekennzeichnet, dass das Pulvergemisch die Reinigungsfunktion und/oder die Deponierfunktion bereitstellt und dass zwischen der Reinigungsfunktion und der Deponierfunktion über die Menge an einem Fluid gewechselt werden kann, mit welchem das Pulvergemisch vermischbar ist. Mit Vorteil ist das Pulvergemisch daher ausgelegt, mit einem Fluid, insbesondere mit Wasser, vermischt zu werden. Erst damit ist der hohe Komfort bei der Behandlung für den Patienten überhaupt erzielbar. So ist es durchaus bekannt, Remineralisierungsfilme mittels eines Pulvers auf Zähne aufzubringen. Diese sind aber nicht mit Wasser vermischbar bzw. wenn doch, so haften diese nur schlecht auf den Zahnoberflächen. Der Wasseranteil ermöglicht eine komfortable Behandlung für den Patienten, da der Mundraum ohne das Wasser beim Aufbringen eines trockenen Pulvers in kürzester Zeit austrocknen würde, was sehr unangenehm für den Patienten ist. Zudem kommt es zu einer Staubentwicklung, welche auch den Behandlungserfolg einschränkt, da der behandelnde Arzt die Zähne nicht mehr sieht bzw. den Überblick verliert, welche Stellen er bereits behandelt hat und welche nicht. Mit Vorteil kann der Pulverstrahl durch den Wasseranteil auch fokussiert werden, da das Wasser das Pulvergemisch während des Fluges zweckmäßigerweise einhüllt. Über den Anteil des Wassers bzw. der Wassermenge im Verhältnis zum Pulvergemisch, kann mit Vorteil zwischen der Reinigungsfunktion und der Deponierfunktion hin- und hergewechselt werden. Dabei sind die Deponierfunktion und die Reinigungsfunktion mit Vorteil nicht getrennt voneinander bereitgestellt. Beide Funktionen werden gleichzeitig durch das Pulvergemisch bereitgestellt, das Pulvergemisch kann aber auch so eingestellt werden, beispielsweise über den zu gemischten Wasseranteil, dass die Deponierfunktion gegenüber der Reinigungsfunktion überwiegt und/oder umgekehrt. Es kann auch nur die Reinigungsfunktion oder nur die Deponierfunktion bereitgestellt werden. Der Erfindung liegt das Prinzip zu Grunde, wonach die Reinigungsfunktion bei höheren Flüssigkeitsmengen überwiegt und die Deponierfunktion bei geringeren Flüssigkeitsmengen.

Bevorzugterweise ist das Pulvergemisch dadurch gekennzeichnet, dass das Deponierpulver ein Phospatpulver ist und dass der Anteil des Phosphatpulvers am Pulvergemisch bevorzugt etwa 0,5 bis 30 % beträgt. Besonders bevorzugt beträgt der Anteil etwa 2 bis 5 %. Mit Vorteil ermöglichen die Anteile das Vermischen mit dem Fluidanteil. Erst dadurch wird ein Haften des Pulvergemisches auf den Zähnen und damit die Bildung eines Remineralisierungsfilms bei gleichzeitig höchstem Komfort für den Patienten möglich.
Erfindungsgemäß ist das Pulvergemisch dadurch gekennzeichnet, dass das Deponierpulver Agglomerate von Nanopartikeln aufweist und dass ein Agglomerat in etwa eine spezifische Oberfläche > 50 m²/g, bevorzugt > 60 m²/g, aufweist. Für die Bildung der Agglomerate sind schwache van-der-Waals-Kräfte verantwortlich.

Erfindungsgemäß ist das Pulvergemisch dadurch gekennzeichnet, dass eine Größe des Agglomerats in einem Bereich von etwa 1 bis 10 µm liegt. Besonders bevorzugt liegt die Größe in einem Bereich von etwa 2 bis 6 µm. Dabei ist mit der Größe ein Durchmesser oder eine maximale Erstreckung des Agglomerats (abhängig von dessen Form) gemeint. Das Deponierpulver weist also Nanopartikel mit einer mittleren Partikelgröße im Bereich von etwa 50 - 500nm, bevorzugt etwa 200nm auf, die sich zu Agglomeraten in dem vorgenannten Größenbereichen zusammenballen.

Bevorzugt liegt ein Molverhältnis von Kalzium zu Phosphat bei den bevorzugten Phosphatpulvern in einem Bereich von etwa 0,5 bis 4, besonders bevorzug in einem Bereich von 1 bis 2,5, ganz besonders bevorzugt ein einem Bereich von etwa 1,5 bis 1,8.

Bevorzugt ist das Pulvergemisch dadurch gekennzeichnet, dass das Deponierpulver Hydroxylapatit, Trikalziumphosphat und/oder Octokalziumphosphat aufweist. Mit anderen Worten ist das bereist angesprochene Phosphatpulver Hydroxylapatit, Trikalziumphosphat und/oder Octokalziumphosphat. Bevorzugt erfolgt eine Kalzinierung des Hydroxylapatis, Trikalziumphosphats und/oder Octokalziumphosphats bei etwa 1200 °C. Es versteht sich, dass das Hydroxylapatit, Trikalziumphosphat und/oder Octokalziumphosphat mit Vorteil in Form der bereits angesprochenen Agglomerate von Nanopartikeln vorliegt.

Zweckmäßigerweise ist das Pulvergemisch dadurch gekennzeichnet, dass das Deponierpulver und/oder das Trägerpulver Träger von Medikamenten und/oder anderen Zusatzstoffen ist. Mit Vorteil kann ein Zusatzstoff beispielsweise ein Farbstoff sein, über welchen die Farbe des aufgebrachten bzw. aufbringbaren Remineralisierungsfilms an die bestehenden Zähne angepasst werden kann. Es versteht sich, dass die Zähne, falls gewünscht, auch farblich verändert, beispielsweise aufgehellt, werden können. Die Zusatzstoffe können Stoffe umfassen, welche die Remineralisierung der Zähne vorantreiben bzw. unterstützen und die Zähne vor weiteren Einflüssen schützen. Vorteilhafterweise können über zumindest einen Zusatzstoff dem Pulvergemisch verschiedene Geschmacksrichtungen gegeben werden. Mit Vorteil kann ein Zusatzstoff auch ein z. B. farbiger Marker sein, welcher dem behandelnden Arzt anzeigt, welche Zahnbereiche bereits behandelt wurden. Der farbige Marker ist zweckmäßigerweise derart ausgelegt, dass er nach einer festlegbaren Zeit seine Farbigkeit verliert, mit anderen Worten sozusagen wieder unsichtbar wird, um die behandelten Zahnbereiche nicht unnatürlich zu verfärben.

Erfindungsgemäß umfasst ein Pulverstrahlgerät, insbesondere zur Anwendung im Dentalbereich, eine Mischeinheit und eine Steuereinheit, wobei die Mischeinheit ausgelegt ist, aus einem Fluidstrom und/oder einem Luftstrom und zumindest einem Pulver ein Fluid-Pulver-Gemisch herzustellen, dadurch gekennzeichnet, dass das Pulverstrahlgerät eine Deponierfunktion aufweist, mittels welcher das Pulverstrahlgerät einen Remineralisierungsfilm auf Zahnoberflächen bilden kann und dass über die Steuereinheit die Bildung des Remineralisierungsfilms beeinflussbar ist. Bei dem Pulver handelt es sich mit Vorteil um das erfindungsgemäße Pulvergemisch.

Zweckmäßigerweise ist die Mischeinheit also ausgelegt, das Pulvergemisch, den Fluidstrom, besonders bevorzugt einen Wasserstrom, und/oder einen Luftstrom zu mischen. Ebenfalls bevorzugt kann die Mischeinheit auch ausgelegt sein, dass erfindungsgemäße Pulvergemisch zu mischen, bevorzugt aus einem Deponierpulver und einem Trägerpulver. Ebenfalls bevorzugt kann die Mischung des Deponierpulvers auch zunächst mit Wasser oder Luft und dann mit dem Trägerpulver erfolgen und/oder umgekehrt. Die Schwierigkeit besteht hierbei grundsätzlich darin, dass das Deponierpulver sehr fein ist und nur schwer verwirbelt werden kann. Technisch einfacher ist es daher, ein bereits fertig gemischtes erfindungsgemäßes Pulvergemisch zu verwenden. Zweckmäßigerweise umfasst das Pulverstrahlgerät ein Handgerät mit einem Anschluss für das Fluid-Pulver-Gemisch. Mit Vorteil umfasst das Pulverstrahlgerät weiter eine stationäre Einheit, wobei die stationäre Einheit einen Pulverbehälter zur Aufnahme des erfindungsgemäßen Pulvergemisches aufweist und mit dem Handgerät über Versorgungsleitungen für Luft, Wasser, Pulvergemisch etc. verbunden ist. Mit Vorteil ist der Pulverbehälter als die Mischeinheit ausgebildet. Alternativ bevorzugt kann das Pulverstrahlgerät auch als ein Handgerät ausgebildet sein, das selbst den Pulverbehälter bzw. die Mischeinheit aufweist und beispielsweise über Versorgungsleitungen für Luft und Wasser mit einer stationären Einheit verbunden ist. Das Fluid-Pulver-Gemisch wird in diesem Fall erst im Handgerät gebildet.

Mit Vorteil ist das Pulverstrahlgerät dadurch gekennzeichnet, dass die Steuereinheit über das Verhältnis des Fluidstroms zum Anteil des Pulvers oder Pulvergemischs das Bilden des Remineralisierungsfilms beeinflusst. Zweckmäßigerweise ist das Pulver das erfindungsgemäße Pulvergemisch, welches ein Trägerpulver aufweist. Je geringer die Steuereinheit den Wasseranteil regelt, desto höher ist die Deponierfunktion. Ist das Trägerpulver ein Reinigungspulver, kann die Steuereinheit durch Erhöhen des Wasseranteils die Reinigungsfunktion erhöhen. Das Umschalten kann mit Vorteil automatisch erfolgen und wird dem Benutzer des Pulverstrahlgeräts angezeigt. Es versteht sich, dass sowohl ein definiertes Umschalten zwischen der Reinigungsfunktion und der Deponierfunktion möglich ist (es wird also entweder nur gereinigt oder nur ein Remineralisierungsfilm aufgebracht). Ebenfalls können aber beide Funktionen gleichzeitig genutzt werden, wobei erfindungsgemäß der Anteil der Reinigungsfunktion zur Deponierfunktion mit Vorteil über den Fluidanteil regulierbar ist. Dabei erfolgt, wie bereits angedeutet, mit Vorteil zunächst die Reinigung und anschließend das Aufbringen des Remineralisierungsfilms. Die hierfür nötigen Zeiten werden bevorzugt automatisch von der Steuereinheit errechnet, welche hierzu zweckmäßigerweise in geeigneter Weise parametrierbar ist, beispielsweise indem die Größe des zu behandelnden Bereichs oder die Anzahl der zu behandelnden Zähne eingegeben werden können. Dem Benutzer wird dann in geeigneter Weise mitgeteilt, wann die Reinigung beendet bzw. wann mit dem Aufbringen des Remineralisierungsfilms begonnen werden kann und wie lange die entsprechenden Bereiche behandelt werden müssen, damit ein ausreichend dicker Reminderalisierungsfilm entstanden ist.

Mit Vorteil kann die Steuereinheit auch automatisch von der Reinigungsfunktion auf die Deponierfunktion umschalten. Zweckmäßigerweise ist der Wasseranteil aber auch direkt vom Benutzer über ein Eingabemittel, beispielsweise einen Drehschalter, ein elektronisches Bedienfeld oder einen entsprechend parametrierten Touchscreen, einstellbar. Es versteht sich, dass die genannten Merkmale und Vorteile auch analog für einen Erhöhung bzw. Erniedrigung des Wasserdrucks gelten, welcher natürlicherweise mit der Wassermenge korreliert. Gleiches gilt für den Luftdruck, welcher die Zusammensetzung des Fluid-Pulver-Gemisches ebenfalls beeinflusst. Mit Vorteil sieht die Steuereinheit auch eine Intervallfunktion vor, wobei diese bewirkt, dass das Fluid-Pulver-Gemisch in bestimmten und festlegbaren Zeitintervallen für die Bildung des Reminieralisierungsfilms ausgedüst wird. Bevorzugt kann dadurch eine sehr gleichmäßige Dicke des Remineralisierungsfilms bereitgestellt werden. So kann das Intervall zweckmäßigerweise derart eingestellt werden, dass so lange ausgedüst wird, bis eine gewünschte Schichtdicke des Remineralisierungsfilms erreicht ist. Der Benutzer hält während dieses Zeitraumes die Düse des Pulverstrahlgeräts, bevorzugt im Wesentlichen an ein und derselben Stelle der Zahnoberfläche. Ist das Intervall zu Ende, weiß der Benutzer, dass er nun die Düse etwas versetzen muss. Auf diese Weise kann der Benutzer sozusagen einen ganzen Zahn bzw. die Zahnoberflächen Schritt für Schritt abfahren und so eine gleichmäßige Schichtdicke erzielen.

Vorteilhafterweise ist das Pulverstrahlgerät dadurch gekennzeichnet, dass die Steuereinheit über Druck, Temperatur und/oder Geschwindigkeit des Fluid-Pulver-Gemisches das Bilden des Remineralisierungsfilms beeinflusst. Das Fluid-Pulver-Gemisch weist während des Fluges eine kinetische Energie auf, welche beim Aufprallen auf der Zahnoberfläche in Wärme umgewandelt wird. Diese Wärme wird mit Vorteil zur Bildung des Remineralisierungsfilms verwendet. Im Nanosekundenbereich können hier Temperaturen von etwa 500 bis 600 °C auftreten. Mit Vorteil können dadurch die Nanopartikel bzw. die Agglomerate des Pulvergemisches miteinander verschmelzen. Weiter mit Vorteil werden die Partikel bzw. die Bestandteile durch die Wärme an den bereits bestehenden Remineralisierungsfilm bzw. an die Zahnoberfläche(n) angelagert. Es versteht sich, dass durch eine Druckerhöhung (des Luftstroms wie auch des Wasserstroms) die kinetische Energie des Fluid-Pulver-Gemisches veränderbar ist, wodurch die Bildung des Remineralisierungsfilms beeinflussbar ist. Dies gilt in gleicher Weise für eine Erhöhung oder Erniedrigung der Temperatur bzw. der Geschwindigkeit des Fluid-Pulver-Gemisches. Zweckmäßigerweise ist das Pulverstrahlgerät dadurch gekennzeichnet, dass das Pulverstrahlgerät eine Reinigungsfunktion aufweist, wobei der Fluidstrom für die Reinigungsfunktion bevorzugt > 20 ml/min ist und wobei der Fluidstrom für die Deponierfunktion bevorzugt etwa 2 bis 10 ml/min beträgt.

Weiter bevorzugt ist das Pulverstrahlgerät dadurch gekennzeichnet, dass zwischen der Deponierfunktion und der Reinigungsfunktion umschaltbar ist. Hierbei ist es vorteilhaft, zwei definierte Wassermengen bzw. Wasseranteile bereitzustellen, zwischen denen wahlweise umgeschaltet werden kann. Beim Umschalten zwischen Reinigungsfunktion und Deponierfunktion kann zusätzlich oder auch alternativ die Pulvermengen- und/oder Druckregelung des Geräts verwendet werden. Ein höherer Luftdruck begünstigt die Reinigungswirkung.

Nach einer bevorzugten Ausführungsform der Erfindung wird zwischen den beiden Betriebszuständen (Reinigung / Deponieren) ausschließlich durch eine verminderte bzw. erhöhte Flüssigkeits- bzw. Fluidmenge umgeschaltet. Das Pulverstrahlgerät kann bevorzugt so beschaffen sein, dass die beiden Betriebszustände voreinstellbar sind und dass der Anwender während einer Behandlung nur zwischen den beiden voreingestellten Betriebszuständen umschaltet, je nachdem welche Funktion er nutzen möchte.

Mit Vorteil ist das Pulverstrahlgerät also insbesondere dadurch gekennzeichnet, dass das Pulverstrahlgerät einen voreinstellbaren Betriebszustand für die Deponierfunktion aufweist und dass das Pulverstrahlgerät einen voreinstellbaren Betriebszustand für die Reinigungsfunktion aufweist und dass das Pulverstrahlgerät zwischen den beiden voreingestellten Betriebszuständen umschaltbar ist.

Weiter mit Vorteil ist das Pulverstrahlgerät dadurch gekennzeichnet, dass das Pulverstrahlgerät eine Heizeinrichtung zur Vorwärmung des Pulvers aufweist. Mit Vorteil ist dadurch die Bildung des Remineralisierungsfilms beeinflussbar. Die genannten Effekte (betreffend die Auswirkungen von Temperaturen) wurden bereits beschrieben. Es versteht sich, dass auch der Fluidstrom vorgewärmt werden kann. Zweckmäßigerweise weist das Pulverstrahlgerät auch eine Lichtquelle auf, welche einen Lichtstrahl bereitstellt, welcher das sich gerade in Bearbeitung befindende Gebiet des Zahnes erleuchtet. Somit weiß der Benutzer, an welcher Stelle er gerade einen Remineralisierungsfilm aufträgt bzw. welche Stelle er reinigt. Damit ist eine besonders gleichmäßige Schichtdicke des Remineralisierungsfilms erreichbar.

Beschrieben ist auch ein geeignetes Mittel zur Remineralisierung von Zahnoberflächen. Dieses Mittel wird unter Verwendung des erfindungsgemäßen Pulvergemischs zuzüglich ggf. weiterer Bestandteile, wie beispielsweise feinteilige Stoffe wie Kieselgel, Bleichmittel, Analgetika, Bakteriozide oder Geschmacksstoffe, die dem Mittel hinzugefügt werden, hergestellt. Weiterhin kann dem Mittel Luft und Wasser hinzugefügt werden, um es mittels des Pulverstrahlgeräts auf die zu bearbeitenden Zahnflächen aufbringen zu können.

Ein Verfahren zur Remineralisierung von Zähnen umfasst die Schritte:
- Bereitstellen eines Pulvers oder eines Pulvergemisches;
- Bereitstellen eines Fluidstroms und/oder eines Luftstroms;
- Mischen des Fluidstroms und/oder des Luftstroms mit dem Pulver oder mit dem Pulvergemisch zu einem Fluid-Pulver-Gemisch;
- Deponieren von Fluid-Pulver-Gemisch auf den Zähnen;
- Steuern des Deponierens durch Einstellen eines Verhältnisses des Fluidstroms zum Pulver oder Pulvergemisch.

Bevorzugt umfasst das Verfahren weiter den Schritt:
- Reinigen der Zähne.

Gemäß der Erfindung wird das Pulvergemischs bevorzugt zusammen mit einem Fluid, bevorzugt Wasser, in einem medizinischen Gerät, insbesondere einem dentalen Pulverstrahlgerät, zur Reinigung und Remineralisierung von Zähnen, verwendet, wobei zwischen der Reinigungsfunktion und der Deponierfunktion des Pulvergemischs über die Menge an Fluid gewechselt werden kann, mit welchem das Pulvergemisch vermischbar ist.

Es versteht sich, dass die genannten Vorteile und Merkmale des erfindungsgemäßen Pulvergemisches auch für das erfindungsgemäße Pulverstrahlgerät, das erfindungsgemäßen Verfahren und die erfindungsgemäße Verwendung gelten sowie auch untereinander und umgekehrt.

## Patentansprüche

1. Pulvergemisch, insbesondere zur Remineralisierung von Zähnen,
**dadurch gekennzeichnet,**
**dass** das Pulvergemisch ein Trägerpulver und ein Deponierpulver aufweist, welches ausgelegt ist, an Zahnoberflächen zu haften, wobei das Deponierpulver ein Phosphatpulver ist, welches zur Remineralisierung der Zähne dient, und Agglomerate von Nanopartikeln aufweist, wobei eine Größe des Agglomerats des Deponierpulvers in einem Bereich von 1 bis 10 µm liegt und wobei das Trägerpulver ein Reinigungspulver ist, welches eine mittlere Teilchengröße von zwischen 10 und 100 µm aufweist wobei die mittlere Teilchengröße des Trägerpulvers größer ist als eine mittlere Teilchengröße des Deponierpulvers und wobei ein Agglomerat des Deponierpulvers eine spezifische Oberfläche > 50 m²/g aufweist.

2. Pulvergemisch nach Anspruch 1,
**dadurch gekennzeichnet,**
**dass** das Reinigungspulver Natriumbikarbonat, Glycin, Alditole, wie z. B. Erythritol oder Kalziumkarbonat ist.

3. Pulvergemisch nach einem der vorhergehenden Ansprüche,
**dadurch gekennzeichnet,**
**dass** der Anteil des Phosphatpulvers am Pulvergemisch 0,5 bis 30 % beträgt.

4. Pulvergemisch nach einem der vorhergehenden Ansprüche,
**dadurch gekennzeichnet, dass** das Deponierpulver Hydroxylapatit, Trikalziumphosphat und/oder Octokalziumphosphat aufweist und
dass das Molverhältnis von Kalzium zu Phosphat bei dem Phosphatpulver in einem Bereich von 0,5 bis 4 liegt.

5. Pulvergemisch nach einem der vorhergehenden Ansprüche,
**dadurch gekennzeichnet,**
**dass** das Deponierpulver und/oder das Trägerpulver Träger von Medikamenten und/oder anderen Zusatzstoffen ist.

6. Pulverstrahlgerät, insbesondere zur Anwendung im Dentalbereich,
umfassend eine Mischeinheit und eine Steuereinheit,
wobei die Mischeinheit ausgelegt ist, aus einem Fluidstrom und/oder einem Luftstrom und zumindest einem Pulvergemisch nach einem der Ansprüche 1 bis 6 ein Fluid-Pulver-Gemisch herzustellen,
**dadurch gekennzeichnet,**
**dass** das Pulverstrahlgerät eine Deponierfunktion aufweist, mittels welcher das Pulverstrahlgerät einen Remineralisierungsfilm auf Zahnoberflächen bilden kann,
**dass** über die Steuereinheit die Bildung des Remineralisierungsfilms beeinflussbar ist, und das Pulverstrahlgerät eine Reinigungsfunktion aufweist und dass das Pulverstrahlgerät zwischen der Deponierfunktion und der Reinigungsfunktion umschaltbar ist, wobei die Steuereinheit über das Verhältnis des Fluidstroms zum Anteil des Pulvergemischs das Bilden des Remineralisierungsfilms beeinflusst, wobei ein Anteil der Reinigungsfunktion zur Deponierfunktion über den Fluidanteil regulierbar ist.

7. Pulverstrahlgerät nach Anspruch 6, **dadurch gekennzeichnet,**
**dass** der Fluidstrom für die Reinigungsfunktion > 20 ml/min ist, und der Fluidstrom für die Deponierfunktion 2 bis 10 ml/min beträgt.

8. Pulverstrahlgerät nach einem der Ansprüche 6-7,
**dadurch gekennzeichnet,**
**dass** das Pulverstrahlgerät einen voreinstellbaren Betriebszustand für die Deponierfunktion aufweist
und **dass** das Pulverstrahlgerät einen voreinstellbaren Betriebszustand für die Reinigungsfunktion aufweist
und **dass** das Pulverstrahlgerät zwischen den beiden voreingestellten Betriebszuständen umschaltbar ist.

9. Pulverstrahlgerät nach einem der Ansprüche 6-8,
**dadurch gekennzeichnet,**
**dass** das Pulverstrahlgerät eine Heizeinrichtung zur Vorwärmung des Pulvers oder Pulvergemischs aufweist.

10. Verwendung eines Pulvergemischs nach einem der Ansprüche 1 - 5, ggf. zusammen mit weiteren feinteiligen Stoffen wie Kieselgel, Bleichmitteln, Analgetika, Bakterioziden und/oder Geschmacksstoffen, zur Herstellung eines Mittels zur Remineralisierung von Zähnen.

## Claims

1. A powder mixture, especially for the remineralization of teeth,
**characterized in that**
the powder mixture comprises a carrier powder and a deposition powder,
which is designed to adhere to tooth surfaces, wherein the deposition powder is a phosphate powder, which is intended for remineralization of the teeth, and which comprises agglomerates of nanoparticles, wherein a size of the agglomerate of the deposition powder is in a range of 1 to 10 µm and the carrier powder is a cleaning powder having an average particle size of between 10 and 100 µm, the average particle size of the carrier powder being larger than an average particle size of the deposition powder and wherein an agglomerate of the deposition powder has a specific surface area > 50 m²/g.

2. The powder mixture according to claim 1,
**characterized in that**
the cleaning powder is sodium bicarbonate, glycine, alditols such as erythritol or calcium carbonate.

3. The powder mixture according to one of the preceding claims,
**characterized in that**
the proportion of phosphate powder in the powder mixture is 0.5 to 30%.

4. The powder mixture according to one of the preceding claims,
**characterized in that** the deposition powder comprises hydroxyapatite, tricalcium phosphate and/or octocalcium phosphate and that the molar ratio of calcium and phosphate in the phosphate powder is in a range of 0.5 to 4.

5. The powder mixture according to one of the preceding claims, **characterized in that**
the deposition powder and/or the carrier powder is a carrier for medications and/or other additives.

6. A powder jet device, especially for use in the dental field,
comprising a mixing unit and a control unit,
wherein the mixing unit is adapted to produce a fluid-powder mixture of a fluid flow and/or an air flow and at least one powder mixture according to one of the claims 1 to 6,
**characterized in that** the powder jet device has a deposition function by which the powder jet device can form a remineralization film on teeth surfaces,
**in that** formation of the remineralization film is controllable by the control unit, and the powder jet device has a cleaning function, and **in that** the powder jet device is switchable between the deposition function and the cleaning function, the control unit controlling formation of the remineralization film by the ratio of the fluid flow and the proportion of the powder mixture, a proportion of the cleaning function to the deposition function being adjustable by the fluid proportion.

7. The powder jet device according to claim 6, **characterized in that** the fluid flow for the cleaning function is > 20 ml/min, and the fluid flow for the deposition function is 2 to 10 ml/min.

8. The powder jet device according to one of the claims 6-7, **characterized in that**
the powder jet device has a presettable operating state for the deposition function and **in that** the powder jet device has a presettable operating state for the cleaning function
and **in that** the powder jet device is switchable between the two preset operating states.

9. The powder jet device according to one of the claims 6-8,
**characterized in that**
the powder jet device comprises heating means for preheating the powder or powder mixture.

10. Use of a powder mixture according to one of claims 1 - 5, optionally together with other finely divided substances such as silica gel, bleaching agents, analgesics, bacteriocides and/or flavouring agents, for the production of an agent for the remineralization of teeth.

## Revendications

1. Mélange de poudres, en particulier pour la reminéralisation des dents, **caractérisé en ce que**
le mélange de poudres comprend une poudre porteuse et une poudre de dépose qui est conçue pour adhérer aux surfaces des dents, la poudre de dépose étant une poudre de phosphate servant à la reminéralisation des dents, et comprenant des agglomérats de nanoparticules, une taille de l'agglomérat de la poudre de dépose étant dans une plage de 1 à 10 µm, et la poudre porteuse étant une poudre de nettoyage présentant une taille moyenne de particules comprise entre 10 et 100 µm, la taille moyenne des particules de la poudre porteuse étant supérieure à une taille moyenne des particules de la poudre de dépose, et un agglomérat de la poudre de dépose présentant une surface spécifique > 50 m²/g.

2. Mélange de poudres selon la revendication 1,
**caractérisé en ce que**
la poudre de nettoyage comprend du bicarbonate de sodium, de la glycine, des alditols tels que l'érythritol, ou du carbonate de calcium.

3. Mélange de poudres selon l'une des revendications précédentes,
**caractérisé en ce que**
la proportion de poudre de phosphate dans le mélange de poudres est de 0,5 à 30 %.

4. Mélange de poudres selon l'une des revendications précédentes,
**caractérisé en ce que**
la poudre de dépose comprend de l'hydroxyapatite, du phosphate tricalcique et/ou du phosphate d'octacalcium, et
**en ce que** dans la poudre de phosphate, le rapport molaire entre le calcium et le phosphate est dans une plage d'environ 0,5 à 4.

5. Mélange de poudres selon l'une des revendications précédentes,
**caractérisé en ce que**
la poudre de dépose et/ou la poudre porteuse est support de médicaments et/ou d'autres additifs.

6. Appareil à jet de poudre, en particulier pour une application dans le domaine dentaire,
comprenant une unité de mélange et une unité de commande,
l'unité de mélange étant conçue pour produire un mélange fluide-poudre à partir d'un débit de fluide et/ou d'un débit d'air et d'au moins un mélange de poudres selon l'une des revendications 1 à 6,
**caractérisé en ce que**
l'appareil à jet de poudre présente une fonction de dépose au moyen de laquelle l'appareil à jet de poudre peut former un film de reminéralisation sur les surfaces des dents,
**en ce que** la formation du film de reminéralisation peut être influencée par l'intermédiaire de l'unité de commande, et l'appareil à jet de poudre présente une fonction de nettoyage, et
**en ce que** l'appareil à jet de poudre peut commuter entre la fonction de dépose et la fonction de nettoyage, l'unité de commande influençant la formation du film de reminéralisation par le biais du rapport entre le débit de fluide et la proportion de mélange de poudre, une proportion de la fonction de nettoyage par rapport à la fonction de dépose étant régulable par le biais de la proportion de fluide.

7. Appareil à jet de poudre selon la revendication 6,
**caractérisé en ce que**
le débit de fluide pour la fonction de nettoyage est > 20 ml/min, et
le débit de fluide pour la fonction de dépose est de 2 à 10 ml/min.

8. Appareil à jet de poudre selon l'une des revendications 6 à 7,
**caractérisé en ce que**
l'appareil à jet de poudre présente un état de fonctionnement préréglable pour la fonction de dépose,
et **en ce que** l'appareil à jet de poudre présente un état de fonctionnement préréglable pour la fonction de nettoyage,
et **en ce que** l'appareil à jet de poudre peut commuter entre les deux états de fonctionnement préréglés.

9. Appareil à jet de poudre selon l'une des revendications 6 à 8,
**caractérisé en ce que**
l'appareil à jet de poudre comprend un dispositif de chauffage pour préchauffer la poudre ou le mélange de poudres.

10. Utilisation d'un mélange de poudres selon l'une des revendications 1 à 5, éventuellement en association avec d'autres substances à particules fines, telles que du gel de silice, des agents de blanchiment, des analgésiques, des bactéricides et/ou des agents aromatiques, pour la préparation d'un agent de reminéralisation des dents.
